(19) Europäisches Patentamt
European Patent Office
Office européen des brevets

(11) Veröffentlichungsnummer : **0 151 792**
**B1**

(12) # EUROPÄISCHE PATENTSCHRIFT

(45) Veröffentlichungstag der Patentschrift : **30.11.88**

(51) Int. Cl.$^4$ : **C 09 B 62/02**

(21) Anmeldenummer : **84116199.5**

(22) Anmeldetag : **22.12.84**

(54) **Umsetzung von Aminen mit heterocyclischen Reaktivkomponenten.**

(30) Priorität : **07.01.84 DE 3400411**

(43) Veröffentlichungstag der Anmeldung : **21.08.85 Patentblatt 85/34**

(45) Bekanntmachung des Hinweises auf die Patenterteilung : **30.11.88 Patentblatt 88/48**

(84) Benannte Vertragsstaaten : **CH DE FR GB LI**

(56) Entgegenhaltungen :

**Keine**

(73) Patentinhaber : **BAYER AG**
**Konzernverwaltung RP Patentabteilung**
**D-5090 Leverkusen 1 Bayerwerk (DE)**

(72) Erfinder : **Wolff, Joachim, Dr.**
**Neuenhauserweg 6**
**D-5060 Bergisch-Gladbach (DE)**
Erfinder : **Wolf, Karlheinz, Dr.**
**Paul-Klee-Strasse 77**
**D-5090 Leverkusen (DE)**
Erfinder : **Wegner, Peter, Dr.**
**Geibelstrasse 9**
**D-5090 Leverkusen (DE)**

**EP 0 151 792 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Umsetzung von primären oder sekundären, aromatische Zentren enthaltenden Aminen in wäßriger Lösung oder Suspension unter Zusatz von säurebindenden Mitteln bei pH 2-12 mit Verbindungen der Formel

$$R^1—X \qquad (1)$$

wobei

$R^1$ = heterocyclischer Reaktivrest

und X = Abgangsgruppe (vorzugsweise Halogen).

Die erfindungsgemäße Umsetzung ist dadurch gekennzeichnet, daß sie unter Zusatz reaktionsbeschleunigender heterogener Katalysatoren aus der Reihe der Metalloxide von Hauptgruppen- oder Übergangsgruppenelementen, Kieselgure, Kieselgele und Kieselsole, festen anorganischen Säuren und Basen des Titans und Wolframs, Oxide der Alkali- und Erdalkalimetalle auf Trägermaterialien, Bleicherden, Tonsile, des Talkums, Sande, Spinelle, synthetischer organischer Kationen- und Anionenaustauscher, Molekularsiebe und anorganischer Ionenaustauscher stattfindet.

Als primäre oder sekundäre, aromatische Zentren enthaltende Amine kommen vorzugsweise solche aus der Reihe der Anthrachinone, Naphthaline, Benzole, Mono-, Dis- oder Trisazofarbstoffe sowie Cu- oder Ni-Phthalocyanine in Frage.

Die reaktionsbeschleunigenden heterogenen Katalysatoren besitzen eine Mindestoberfläche von 0,001 $m^2/g$, bevorzugt $\geq 0{,}1\ m^2/g$ und insbesondere $> 10\ m^2/g$.

Aus US-A 3 492 286 (Spalte 3, Zeile 30) ist bereits bekannt, die Umsetzung von Aminen mit Reaktivkomponenten durch den Zusatz von Netz- und Emulgiermitteln zu beschleunigen.

Im Unterschied dazu und zu den Säuren und Basen in homogenen flüssigen Systemen besitzen die festen sauren und basischen Katalysatoren Zentren unterschiedlicher Stärke und Art, so wurden z. B. an $Al_2O_3$-Oberflächen sowohl saure als auch basische Zentren nachgewiesen. In dem erfindungsgemäßen Verfahren haben sich gerade jene Katalysatortypen als besonders wirkungsvoll erwiesen.

Als organische Ionenaustauscher gelten kettenförmige und teilweise vernetzte Polymere, deren Moleküle spezifische funktionelle ionogene Gruppen enthalten. Desweiteren können Verwendung finden heterogene Übergangsmetall-Komplexe (Metallionen koordinativ verbunden mit festen organischen (Polymere) oder anorganischen Trägern durch koordinationsfähige Gruppen des Trägers), bi- und multifunktionelle Katalysatoren (z. B. Pt/Aluminiumsilikat) sowie Aktivkohlen bzw. Mischungen der genannten Systeme. Unter heterocyclischen Reaktivresten $R^1$ in der mit der Verbindung (2) nach dem neuen Verfahren umzusetzenden Molekül $R^1$-X (X = Halogen) werden verstanden :

Substituenten, die eine oder mehrere reaktive Gruppen oder abspaltbare substituenten aufweisen, welche beim Aufbringen der Farbstoffe auf Cellulosematerialien in Gegenwart säurebindender Mittel, gegebenenfalls unter Einwirkung von Wärme mit den Hydroxylgruppen der Cellulose oder beim Aufbringen auf Superpolyamidfasern, wie Wolle, mit den NH-Gruppen dieser Fasern unter Ausbildung kovalenter Bindungen zu reagieren vermögen.

Bevorzugt werden nach dem erfindungsgemäßen Verfahren Verbindungen der Formel

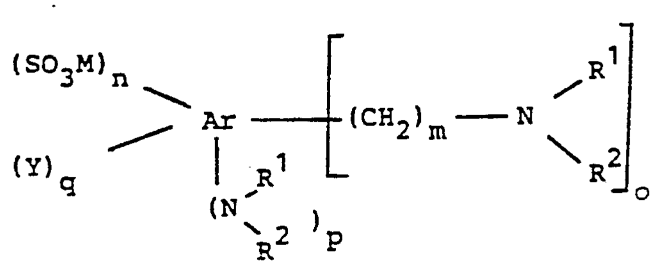

(3)

hergestellt, wobei

Ar = Rest der Benzol- oder Naphthalinreihe oder eines chromophoren Systems der Mono-, Dis- oder Trisazoreihe, der Anthrachinon-, oder Oxazin-Formazanreihe oder der Reihe der Cu- oder Ni-Phthalocyaninfarbstoffe

Y = Substituent

M = Wasserstoff oder ein Metalläquivalent

$R^1$ = heterocyclischer Reaktivrest

$R^2$ = H oder $C_1$-$C_4$-Alkyl

m, q = ganze Zahl von 0 bis 6

n = ganze Zahl von 1 bis 5

o, p = 0 oder 1,

wobei o + p = 1.

Als Substituenten Y kommen vorzugsweise in Frage:
Alkyl (vorzugsweise $C_1$-$C_4$-Alkyl, insbesondere Methyl),
Alkoxy (vorzugsweise $C_1$-$C_4$-Alkoxy, insbesondere Methoxy),
Hydroxy, Carboxy, Amino, $NO_2$, acyliertes Amino (beispielsweise Acetylamino, Benzoylamino), Halogen (insbesondere Chlor), Ureido, Sulfonylreste.

Falls q = 2, können die einzelnen Substituenten Y gleich oder verschieden sein.

Als Metalläquivalente M kommen vorzugsweise in Frage: $Na^+$, $K^+$, $NH_4^+$, $Li^+$.

$R^2$ = $C_1$-$C_4$-Alkyl steht vorzugsweise für $R^2$ = Methyl.

Die Verbindungen der Formel (3) werden erfindungsgemäß erhalten, indem man Ausgangsverbindungen der allgemeinen Formel

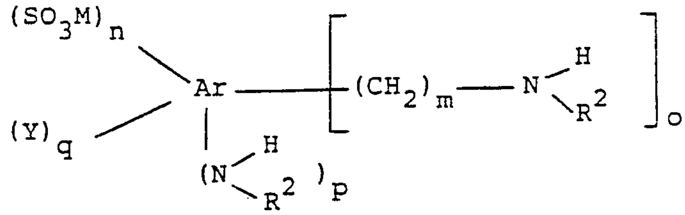

(2)

wobei M, $R^2$, Ar, Y, m, n, o, p, q die unter Formel (3) angegebene Bedeutung besitzen mit der heterocyclischen Reaktivkomponente der Formel

$$R^1—X \qquad (1)$$

worin

$R^1$ = heterocyclischer Reaktivrest

X = Abgangsgruppe (vorzugsweise Cl oder F)

in wäßriger Lösung oder Suspension, die gegebenenfalls konzentrationserhöhende Zusatzstoffe enthält, unter Zusatz von säurebindenden Mitteln bei pH 2-12 in Gegenwart heterogener Katalysatoren umsetzt, wobei abhängig von der Natur der Ausgangsverbindungen und/oder der Zusatzstoffe, Zwischenprodukte, Farbstoffendprodukte oder auch bereits gebrauchsfertige Farbstoffpräparationen erhalten werden.

Es ist bereits bekannt, Verbindungen der Formel (3) ohne Zusatz von heterogenen Katalysatoren in wäßriger Lösung bzw. Suspension herzustellen. Es gibt aber eine Reihe aminohaltiger aromatischer Molekeln, insbesondere Azofarbstoffe, die nach den üblichen Verfahren nur mit sehr geringer Ausbeute mit der heterocyclischen Reaktivkomponente $R^1$-X umgesetzt werden können. So liefert z. B. der Azofarbstoff der Formel (4)

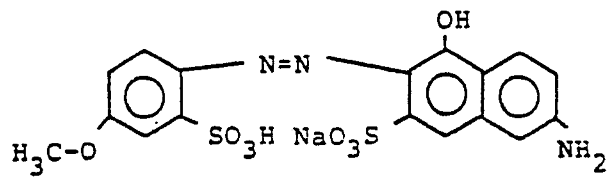

(4)

in einer 15 %igen Lösung mit 2,4,6-Trifluor-5-chlorpyrimidin (FCP) nur ca. 60 Gew.-% Umsetzungsprodukt. Es wurde nun in hohem Maße überraschend gefunden, daß z. B. unter Zusatz geringer Mengen an pyrogenen oder Fällungs-Kieselsäuren als heterogener Katalysator bei sonst identischen Reaktionsbedingungen, 95 % des Azofarbstoffs (4) mit Trifluorchlorpyrimidin umgesetzt werden. Als weiteres Beispiel kann gezeigt werden, daß die Kondensation von I-Säure (5)

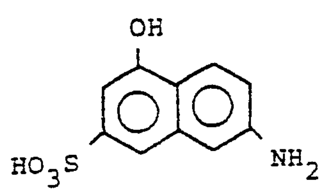

(5)

mit 2,4,6-Trifluor-5-chlorpyrimidin nach bekannter Verfahrensweise mit zunehmender Konzentration in Wasser langsamer und unvollständiger verläuft. Werden dagegen z. B. geringe Mengen an Bleicherde als heterogener Katalysator zugegeben, so erhält man auch in einer wäßrigen Lösung mit 20 Gew.-% I-Säure (5) nach ca. 2 Stunden ca. 95 % Umsetzungsprodukt.

Der Vorteil des neuen erfindungsgemäßen Verfahrens ist, daß viele Reaktionen wesentlich schneller

und vollständiger verlaufen. Es hat sich dabei als in hohem Maße überraschend gezeigt, daß dies bei vergleichenden Reaktionsbedingungen lediglich durch Zugabe von heterogenen Katalysatoren gegebenenfalls in Gegenwart konzentrationserhöhender Zuatzstoffe erreicht wird. Auf Gewicht Ausgangsamin bezogen, werden die entsprechend genannten heterogenen Katalysatoren ab 0,001 Gew.-% eingesetzt, bevorzugt ab 0,01 Gew.-%.

Als Beispiele für Ausgangsverbindungen der Formel (2) seien aufgeführt :

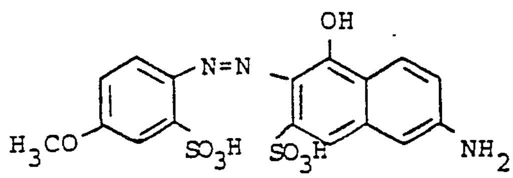

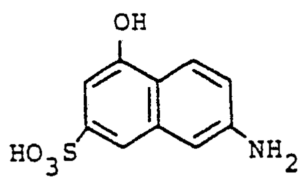
,
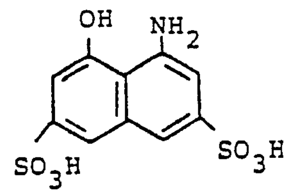

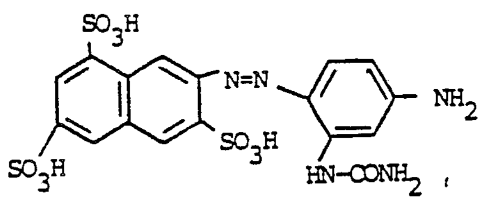
,

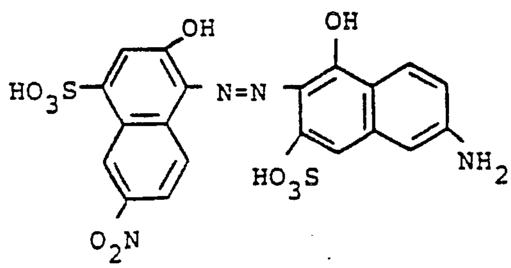

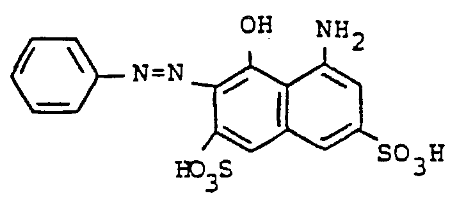

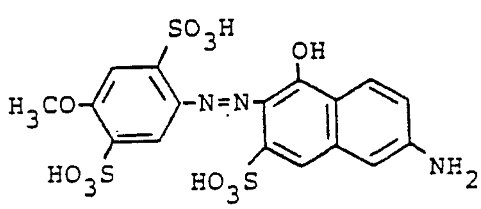

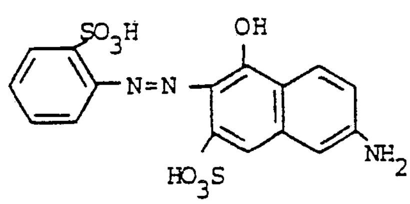

(Fortsetzung)

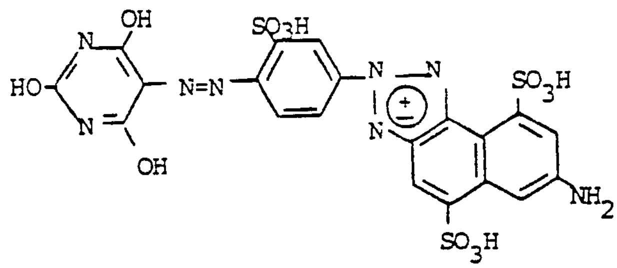

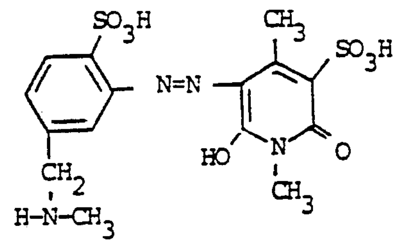

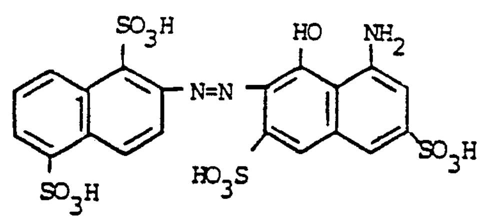

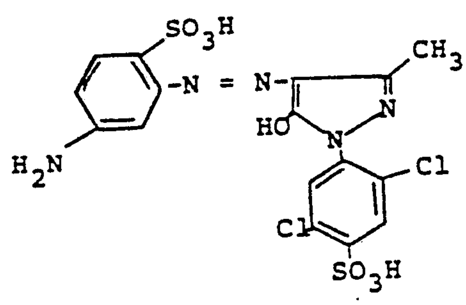

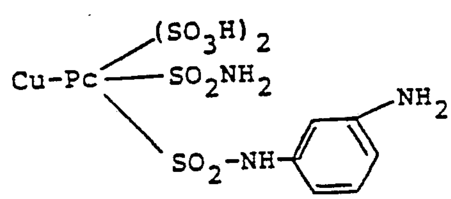

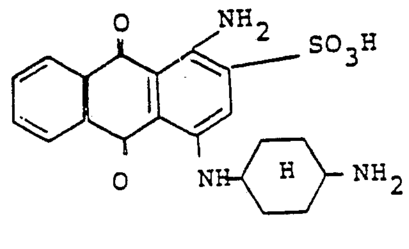
,
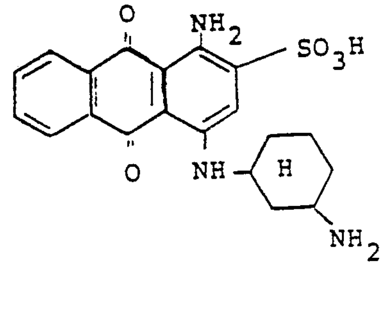

(Fortsetzung)

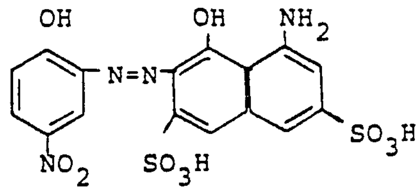

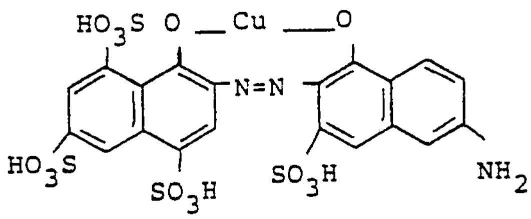

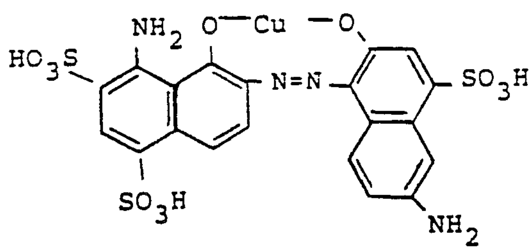

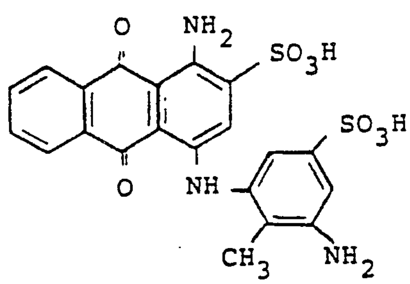

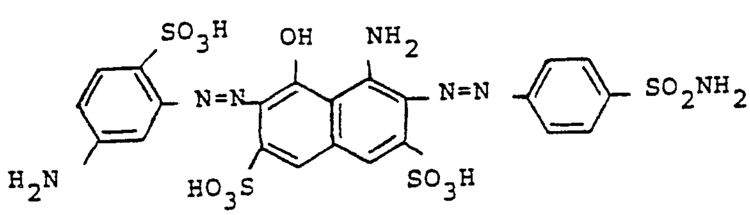

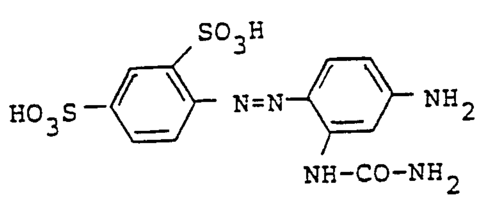

(Fortsetzung)

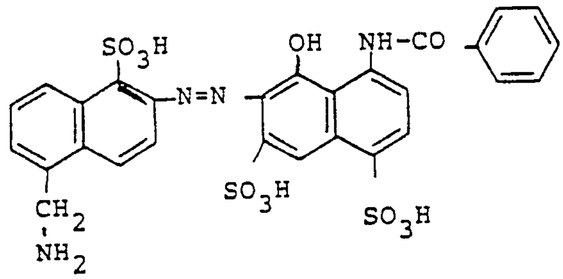

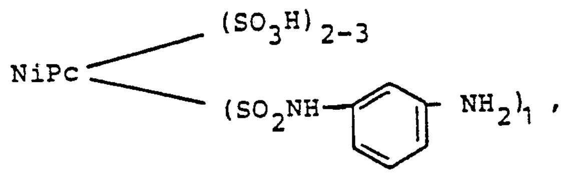

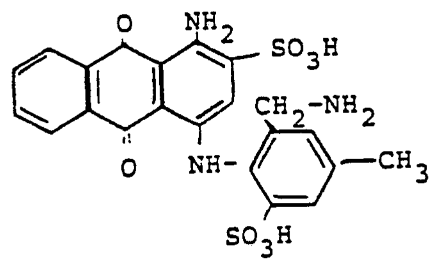

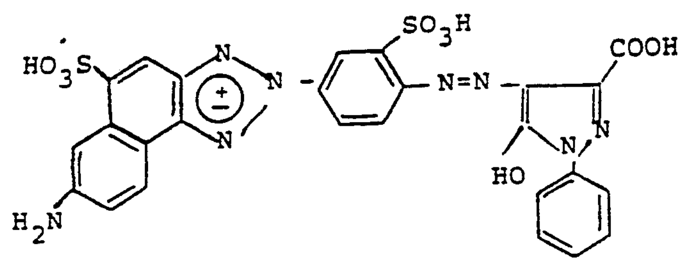

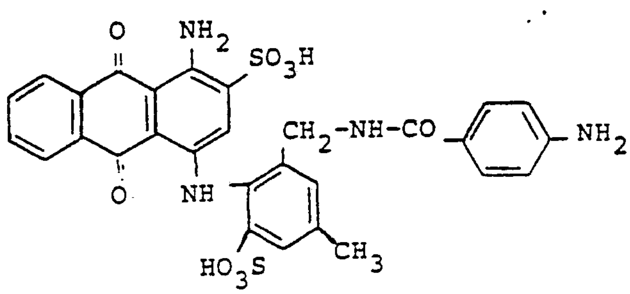

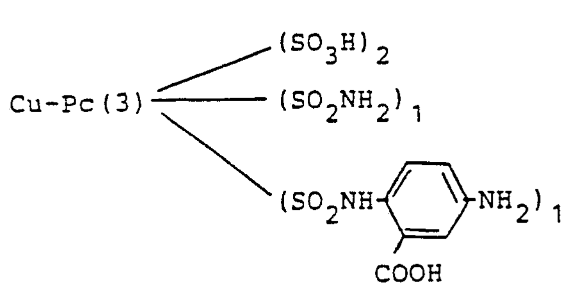

Bevorzugt werden als Verbindungen der Formel (2) Hydroxyaminonaphthalinsulfonsäuren oder Azofarbstoffe auf Basis dieser Hydroxyaminonaphthalinsulfonsäuren (als Kupplungskomponenten) eingesetzt. Besonders bevorzugt wird als Verbindung der Formel (2) 1-Hydroxy-3-sulfo-6-aminonaphthalin oder ein Azofarbstoff auf Basis von 1-Hydroxy-3-sulfo-6-aminonaphthalin eingesetzt.

Als Reaktivkomponenten der Formel (1) werden vorzugsweise solche der folgenden heterocyclischen Systeme eingesetzt :

Halogentriazine, Halogenpyrimidine, Halogenpyridazine, Chinolinderivate, Pyridazon-Verbindungen, Halogenchinoxaline, Halogenphthalazine, Halogenchinazoline, Benzthiazolverbindungen, Benzoxazol-Derivate und andere.

Aus der großen Zahl der zur Verfügung stehenden Verbindungen seien hier beispielsweise erwähnt :

Trihalogen-symmetrische-triazine, wie Cyanurchlorid und Cyanurbromid, Dihalogen-monoamino- und monosubstituierteaminosymmetrische triazine, wie 2,6-Dichlor-4-aminotriazin, 2,6-Dichlor-4-methylaminotriazin, 2,6-Dichlor-4-ethylaminotriazin, 2,6-Dichlor-4-oxethylaminotriazin, 2,6-Dichlor-4-phenylaminotriazin, 2,6-Dichlor-4-(o-, m- oder p-sulfophenyl)-aminotriazin, 2,6-Dichlor-4-(2',3'-, 2',4'-, 3',4'- oder 3',5'-disulfophenyl)-aminotriazin, Dihalogen-alkoxy- und aryloxy-symmetrische-triazine, wie 2,6-Dichlor-4-methoxytriazin, 2,6-Dichlor-4-ethoxytriazin, 2,6-Dichlor-4-phenoxytriazin, 2,6-Dichlor-4- (o-, m- oder p-sulfophenyl)-oxytriazin, Dihalogen-alkylmercapto- und -arylmercapto symmetrische-triazine, wie 2,6-Dichlor-4-ethylmercapto-triazin, 2,6-Dichlor-4-phenylmercaptotriazin, 2,6-Dichlor-4-(p-methylphenyl)-mercaptotriazin, 2,4,6-Trifluortriazin, 2,4-Difluor-6-(o-, m-, p-methyl-phenyl)-amino-triazin, 2,4-Difluor-6-(o-, m-, p-sulfophenyl)-amino-triazin, 2,4-Difluor-6-methoxy-triazin, 2,4-Difluor-6-(2',5'-disulfophenyl)-amino-triazin, 2,4-Difluor-6-(6'-sulfonaphthyl-2')-amino-triazin, '2,4-Difluor-6-(o-, m-, p-ethylphenyl)-amino-triazin, 2,4-Difluor-6-(o-, m-, p-chlorphenyl)-amino-triazin, 2,4-Difluor-6-(o-, m-, p-methoxyphenyl)-amino-triazin, 2,4-Difluor-6-(2'-methyl-5'-sulfophenyl)-amino-triazin, 2,4-Difluor-6-(2'-methyl-4'-sulfophenyl)-amino-triazin, 2,4-Difluor-6-(2'-chlor-4'-sulfophenyl)-amino-triazin, 2,4-Difluor-6-(2'-chlor-5'-sulfophenyl)-amino-triazin, 2,4-Difluor-6-(2'-methoxy-4'-sulfophenyl)-amino-triazin, 2,4-Difluor-6-(o-, m-, p-carboxyphenyl)-amino-triazin, 2,4-Difluor-6-(N-methyl-phenyl)-amino-triazin, 2,4-Difluor-6-(N-ethyl-phenyl)-amino-triazin, 2,4-Difluor-6-(N-isopropyl-phenyl)-amino-triazin, 2,4-Difluor-6-cyclohexylamino-triazin, 2,4-Difluor-6-morpholino-triazin, 2,4-Difluor-6-piperidino-triazin, 2,4-Difluor-6-benzylamino-triazin, 2,4-Difluor-6-N-methylbenzylamino-triazin, 2,4-Difluor-6-β-phenylethyl-amino-triazin, 2,4-Difluor-6-(X-sulfo-benzyl)-amino-triazin, 2,4-Difluor-6-(2',4'-disulfophenyl)-amino-triazin, 2,4-Difluor-6-(3',5'-disulfophenyl)-amino-triazin, 2,4-Difluor-6-(2'-carboxy-4'-sulfo-phenyl)-amino-triazin, 2,4-Difluor-6-(2',5'-disulfo-4-methoxyphenyl)-amino-triazin, 2,4-Difluor-(2'-methyl-4',6'-disulfophenyl)-amino-triazin, 2,4-Difluor-(6',8'-disulfonaphthyl-(2'))-amino-triazin, 2,4-Difluor-(4',8'-disulfonaphthyl-(2'))-amino-triazin, 2,4-Difluor-(4',6',8'-trisulfo-naphthyl-(2'))-amino-triazin, 2,4-Difluor-(3',6',8'-trisulfonaphthyl-(2'))-amino-triazin, 2,4-Difluor-(3',6'-disulfonaphthyl-(1'))-amino-triazin, 2,4-Difluor-6-amino-triazin, 2,4-Difluor-6-methylaminotriazin, 2,4-Difluor-6-ethylamino-triazin, 2,4-Difluor-6-methoxyethoxy-triazin, 2,4-Difluor-6-methoxyethylamino-triazin, 2,4-Difluor-6-dimethylamino-triazin, 2,4-Difluor-6-diethylamino-triazin, 2,4-Difluor-6-iso-propylamino-triazin, Tetrahalogenpyrimidine, wie Tetrachlor-, Tetrabrom- oder Tetrafluor-pyrimidin, 2,4,6-Trihalogenpyrimidine, wie 2,4,6-Trichlor-, -Tribrom- oder -Trifluor-pyrimidin, Dihalogenpyrimidine, wie 2,4-Dichlor-, -Dibrom- oder -Difluorpyrimidin ; 2,4,6-Trichlor-5-nitro- oder -5-methyl- oder -5-carbomethoxy- oder -5-carboethoxy- oder -5-carboxymethyl- oder -5-mono-, -di- oder -trichlormethyl- oder -5-carboxy- oder -5-sulfo- oder -5-cyano- oder -5-vinylpyrimidin, 2,4-Difluor-6-methylpyrimidin, 2,6-Difluor-4-methyl-5-chlorpyrimidin, 2,4-Difluor-pyrimidin-5-ethylsulfon, 2,6-Difluor-4-chlorpyrimidin, 2,4,6-Trifluor-5-chlorpyrimidin, 2,6-Difluor-4-methyl-5-brompyrimidin, 2,4-Difluor-5,6-dichlor- oder -dibrompyrimidin, 4,6-Difluor-2,5-dichlor- oder -dibrompyrimidin, 2,6-Difluor-4-brompyrimidin, 2,4,6-Trifluor-5-brompyrimidin, 2,4,6-Trifluor-5-chlormethylpyrimidin, 2,4,6-Trifluor-5-nitropyrimidin, 2,4,6-Trifluor-5-cyanpyrimidin, 2,4,6-Trifluorpyrimidin-5-carbonsäurealkylester oder -5-carbonsäureamide, 2,6-Difluor-5-methyl-4-chlorpyrimidin, 2,6-Difluor-5-chlorpyrimidin, 2,4,6-Trifluor-5-methylpyrimidin, 2,4,6-Trifluor-5-methylpyrimidin, 2,4,5-Trifluor-6-methylpyrimidin, 2,4-Difluor-5-nitro-6-chlorpyrimidin, 2,4-Difluor-5-cyanpyrimidin, 2,4-Difluor-5-methylpyrimidin, 6-Trifluormethyl-5-chlor-2,4-difluorpyrimidin, 6-Phenyl-2,4-difluorpyrimidin, 6-Trifluormethyl-2,4-difluorpyrimidin, 5-Trifluormethyl-2,4,6-trifluorpyrimidin, 2,4-Difluor-5-nitro-pyrimidin, 2,4-Difluor-5-trifluormethylpyrimidin, 2,4-Difluor-5-methylsulfonyl-pyrimidin, 2,4-Difluor-5-carbon-amido-pyrimidin, 2,4-Difluor-5-carbomethoxy-pyrimidin, 2,4-Difluor-6-trifluormethylpyrimidin, 2,4-Difluor-6-carbonamido-pyrimidin, 2,4-Difluor-6-carbomethoxy-pyrimidin, 2,4-Difluor-6-phenyl-pyrimidin, 2,4-Difluor-6-cyanpyrimidin, 2,4,6-Trifluor-5-methylsulfonyl-pyrimidin, 2,4-Difluor-5-sulfonamido-pyrimidin, 2,4-Difluor-5-chlor-6-carbomethoxy-pyrimidin, 5-Trifluormethyl-2,4-difluorpyrimidin, 2,4-Dichlorpyrimidin-5-carbonsäurechlorid, 2,4,6-Trichlorpyrimidin-5-carbonsäurechlorid, 2-Methyl-4-chlorpyrimidin-5-carbonsäurechlorid, 2-Chlor-4-methylpyrimidin-5-carbonsäurechlorid, 2,6-Dichlorpyrimidin-4-carbonsäurechlorid ; Pyrimidin-Reaktivkomponenten mit abspaltbaren Sulfonylgruppen, wie 2-Carboxymethylsulfonyl-4-chlorpyrimidin, 2-Methylsulfonyl-4-chlor-6-methylpyrimidin, 2,4-Bis-methyl-sulfonyl-6-methylpyrimidin, 2,4-Bis-phenylsulfonyl-5-chlor-6-methylpyrimidin, 2,4,6-Trismethylsulfonylpyrimidin, 2,6-Bis-methylsulfonyl-4,5-dichlorpyrimidin, 2,4-Bis-methylsulfonylpyrimidin-5-sulfonsäurechlorid, 2-Methylsulfonyl-4-chlorpyrimidin, 2-Phenylsulfonyl-4-chlorpyrimidin, 2,4-Bis-trichlormethylsulfonyl-6-methylpyrimidin, 2,4-Bis-methylsulfonyl-5-chlor-6-methylpyrimidin, 2,4-Bis-methylsulfonyl-5-brom-6-methylpyrimidin, 2-Methylsulfonyl-4,5-dichlor-6-methylpyrimidin, 2-Methylsulfonyl-4,5-dichlor-6-chlormethylpyrimidin, 2-Methylsulfonyl-4-chlor-6-me-

thylpyrimidin-5-sulfonsäurechlorid, 2-Methylsulfonyl-4-chlor-5-nitro-6-methylpyrimidin, 2,4,5,6-Tetramethylsulfonyl-pyrimidin, 2-Methylsulfonyl-4-chlor-5,6-dimethylpyrimidin, 2-Ethylsulfonyl-4,5-dichlor-6-methylpyrimidin, 2-Methylsulfonyl-4,6-dichlorpyrimidin, 2,4,6-Tris-methylsulfonyl-5-chlorpyrimidin, 2-Methylsulfonyl-4-chlor-6-carboxypyrimidin, 2-Methylsulfonyl-4-chlorpyrimidin-5-sulfonsäure, 2-Methylsulfonyl-4-chlor-6-carbomethoxypyrimidin, 2-Methylsulfonyl-4-chlor-pyrimidin-5-carbonsäure, 2-Methylsulfonyl-4-chlor-5-cyan-6-methoxypyrimidin, 2-Methylsulfonyl-4,5-dichlorpyrimidin, 4,6-Bis-methylsulfonylpyrimidin, 4-Methylsulfonyl-6-chlorpyrimidin, 2-Sulfoethylsulfonyl-4-chlor-6-methylpyrimidin, 2-Methylsulfonyl-4-chlor-5-brompyrimidin, 2-Methylsulfonyl-4-chlor-5-brom-6-methylpyrimidin, 2,4-Bis-methylsulfonyl-5-chlorpyrimidin, 2-Phenylsulfonyl-4,5-dichlorpyrimidin, 2-Phenylsulfonyl-4,5-dichlor-6-methylpyrimidin, 2-Carboxymethylsulfonyl-4,5-dichlor-6-methylpyrimidin, 2-(2'- oder 3'- oder 4'-Carboxyphenylsulfonyl)-4,5-dichlor-6-methylpyrimidin, 2,4-Bis-(2' oder 3' oder 4'-Carboxyphenylsulfonyl)-5-chlor-6-methylpyrimidin, 2-Methylsulfonyl-6-chlorpyrimidin-4- oder -5-carbonsäurechlorid, 2-Ethylsulfonyl-6-chlorpyrimidin-4- oder -5-carbonsäurechlorid, 2,6-Bis-(methylsulfonyl)-pyrimidin-4-carbonsäurechlorid, 2-Methylsulfonyl-6-methyl-4-chlor- oder -4-brompyrimidin-5-carbonsäurechlorid oder -bromid, 2,6-Bis-(methylsulfonyl)-4-chlorpyrimidin-5-carbonsäurechlorid ; weitere Reaktivkomponenten der heterocyclischen Reihe mit reaktiven Sulfonylsubstituenten sind beispielsweise 3,6-Bis-phenylsulfonyl-pyridazin, 3-Methylsulfonyl-6-chlorpyridazin, 3,6-Bis-trichlormethylsulfonylpyridazin, 3,6-Bis-methylsulfonyl-4-methylpyridazin, 2,5,6-Tris-methylsulfonylpyrazin, 2,4-Bis-methylsulfonyl-1,3,5-triazin, 2,4-Bis-methylsulfonyl-6-(3'-sulfophenylamino)-1,3,5-triazin, 2,4-Bis-methylsulfonyl-6-N-methylanilino-1,3,5-triazin, 2,4-Bis-methylsulfonyl-6-phenoxy-1,3,5-triazin, 2,4-Bis-methylsulfonyl-6-trichlorethoxy-1,3,5-triazin, 2,4,6-Trisphenylsulfonyl-1,3,5-triazin, 2,4-Bis-methylsulfonylchinazolin, 2,4-Bis-trichlormethylsulfonylchinolin, 2,4-Bis-carboxymethylsulfonylchinolin, 2,6-Bis-(methylsulfonyl)-pyridin-4-carbonsäurechlorid und 1-(4'-Chlorcarbonylphenyl oder 2'-chlorcarbonylethyl)-4,5-bis-methylsulfonyl-pyridazin-(6) ; weitere heterocyclische Reaktivkomponenten mit beweglichem Halogen sind unter anderem 2- oder 3-Monochlorchinoxalin-6-carbonsäurechlorid oder -6-sulfonsäurechlorid, 2- oder 3-Monobromchinoxalin-6-carbonsäurebromid oder -6-sulfonsäurebromid, 2,3-Dichlorchinoxalin-6-carbonsäurechlorid oder -6-sulfonsäurechlorid, 2,3-Dibromchinoxalin-6-carbonsäurebromid oder -6-sulfonsäurebromid, 1,4-Dichlorphthalazin-6-carbonsäurechlorid oder -6-sulfonsäurechlorid sowie die entsprechenden Bromverbindungen, 2,4-Dichlorchinazolin-6-oder -7-carbonsäurechlorid oder -7-sulfonsäurechlorid sowie die entsprechenden Bromverbindungen, 2-oder 3- oder 4-(4',5'-Dichlorpyridazin-6'-yl-1')-phenylsulfonsäurechlorid oder -carbonsäurechlorid sowie die entsprechenden Bromverbindungen, β-(4',5'-Dichlorpyridazon-6'-yl-1')-ethylcarbonsäurechlorid, 2-Chlorchinoxalin-3-carbonsäurechlorid und die entsprechenden Bromverbindungen, N-Methyl-N-(2,4-dichlortriazinyl-6)-carbamidsäurechlorid, N-Methyl-N-(2-chlor-4-methyl-amino-triazinyl-6)-carbamidsäurechlorid, N-Methyl-N-(2-chlor-4-dimethylamino-triazinyl-6)-carbamidsäurechlorid, N-Methyl- oder N-Ethyl-N-(2,4-dichlortriazinyl-6)-aminoacetylchlorid, N-Methyl-, N-Ethyl- oder N-Hydroxyethyl-N-(2,3-dichlorchinoxalin-6-sulfonyl oder -6-carbonyl)-aminoacetylchlorid und die entsprechenden Bromderivate, ferner 2-Chlorbenzthiazol-5- oder -6-carbonsäurechlorid oder -5- oder -6-sulfonsäurechlorid und die entsprechenden Bromverbindungen, 2-Arylsulfonyl- oder 2-Alkylsulfonyl-benzthiazol-5- oder -6-carbonsäurechloride oder -5- oder -6-sulfonsäurechloride, wie 2-Methylsulfonyl- oder 2-Ethyl-sulfonyl- oder 2-Phenylsulfonyl-benzthiazol-5- oder -6-sulfonsäurechlorid oder -5- oder -6-carbonsäurechlorid sowie die entsprechenden in ankondensierten Benzolring Sulfonsäuregruppen enthaltenden 2-Sulfonylbenzthiazol-Derivate, 3,5-Bis-methylsulfonyl-isothiazol-4-carbonsäurechlorid, 2-Chlor-benzoxazol-5- oder -6-carbonsäurechlorid oder -sulfonsäurechlorid sowie die entsprechenden Bromderivate, 2-Chlorbenzimidazol-5- oder -6-carbonsäurechlorid oder -sulfonsäurechlorid sowie die entsprechenden Bromderivate, 2-Chlor-1-methylbenzimidazol-5- oder -6-carbonsäurechlorid oder -sulfonsäurechlorid sowie die entsprechenden Bromderivate, 2-Chlor-4-methylthiazol-(1,3)-5-carbonsäurechlorid (-4-) oder -5-sulfonsäurechlorid, 2-Chlorthiazol-4- oder -5-sulfonsäurechlorid und die entsprechenden Bromderivate.

Im Rahmen der vorliegenden Erfindung bevorzugt hergestellte Verbindungen enthalten vorzugsweise Reaktivgruppen folgender Systeme :

Mono- oder Dihalogen-symmetrische Triazinylreste, Mono-, Di- oder Trihalogenpyrimidinylreste oder halogensubstituierte Chinoxalinylcarbonylreste.

Insbesondere bevorzugt sind folgende Reaktivgruppen der Formel (6), (7) und (8) :

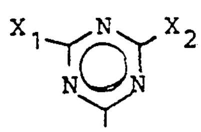

(6)

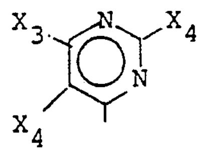

(7)

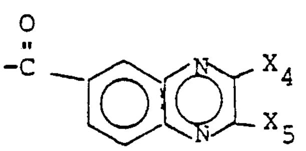

(8)

wobei in den Formeln (6), (7) und (8) gilt :

$X_1$ = F

$X_2$ = Cl, F, $NH_2$, $NHR^3$, $OR^3$, $CH_2R^3$, $SR^3$

$X_3$ = Cl, F, $CH_3$

$X_4$ = Cl, F und

$X_5$ = Cl, F, $CH_3$

und wobei

$R^3$ = Alkyl (insbesondere gegebenenfalls durch OH, $SO_3H$, COOH substituiertes $C_1$-$C_4$-Alkyl), Aryl (insbesondere gegebenenfalls durch $SO_3H$, $C_1$-$C_4$-Alkyl, $C_1$-$C_4$-Alkoxy substituiertes Phenyl), Aralkyl (insbesondere gegebenenfalls durch $SO_3H$, $C_1$-$C_4$-Alkyl, $C_1$-$C_4$-Alkoxy substituiertes Benzyl).

Farbstoffe, welche die obengenannten Reaktivgruppensysteme enthalten sind beispielsweise aus folgenden Publikationen bekannt :

US-PS 3 377 336, US-PS 3 527 760

GB-PS 1 169 254, US-PS 3 669 951

DE-PS 1 644 208, GB-PS 1 188 606

DE-OS 2 817 780, ES-PS 479 771

Als säurebindendes Mittel bei der Umsetzung der Komponente $R^1$-X (3) mit der Verbindung (2) kommen vorzugsweise Alkalien oder Ammoniumsalze wie NaOH, LiOH, KOH, Ca $(OH)_2$, $Na_2CO_3$, $NaHCO_3$, $Li_2CO_3$, $(NH_4)_2CO_3$, $NH_4HCO_3$ in Frage. Mit Hilfe der säurebindenden Mittel wird auf pH 2-12, bevorzugt 4-10 abgestumpft. Wird bei der Umsetzung der Verbindung (2) mit der Reaktivkomponente $R^1$-X HF frei, so ist es für den Fall, daß man in konzentrierten wäßrigen Medien arbeitet vorteilhaft, als säurebindende Mittel bas. $Li^+$ oder $Ca^{2+}$-Salze zu verwenden.

Falls beabsichtigt wird, direkt gebrauchsfertige Flüssigpräparationen herzustellen, so können als konzentrationserhöhende Zusatzstoffe bei dem neuen Herstellungsverfahren z. B. Verwendung finden : cyclische Ether wie Tetrahydrofuran, Ethylencarbonat, Propylencarbonat und/oder niedere aliphatische oder cyclische Amide, wie ε-Caprolactam oder N-Alkyl-pyrrolidone, N-Vinylpyrrolidone und deren Polymere und/oder aliphatische Sulfoxide und/oder schwefelhaltige Verbindungen wie Dimethylsulfon, Diethylsulfon und/oder hydrotrope Verbindungen wie Thioharnstoff, Harnstoff, sowie die dazu gehörigen Derivate, besonders bevorzugt sind N,N'-Dimethylharnstoff, ε-Caprolactam und Dimethylsulfon.

Als gegebenenfalls zugesetzte Puffersubstanzen kommen vorzugsweise solche in Frage, die im Bereich pH 6,5-9 puffern. Beispielhaft seien die folgenden Puffersysteme genannt :

1. Monokaliumdihydrogenphosphat/Dikaliumhydrogenphosphat-Mischungen
2. Borsäure mit Alkalihydroxid auf pH 7-8,5 gestellt
3. Citronensäure mit Alkalihydroxid auf pH 6-8 gestellt
4. $HCO_3^-/CO_3^{2-}$-Mischungen auf pH 7-8,5 gestellt bzw. Mischungen von 1 bis 4.

Die nach dem erfindungsgemäßen Verfahren hergestellten Verbindungen der Formel (3) sind entweder wertvolle Zwischenverbindungen für die Synthese von Reaktivfarbstoffen oder Reaktivfarbstoffe selbst. Die nach dem erfindungsgemäßen Verfahren entweder direkt erhaltenen Reaktivfarbstoffe oder erst nach Kupplung eines diazotierten Amins auf Verbindung (3) gewonnenen Reaktivfarbstoffe können auf verschiedene Art weiterverarbeitet werden. Für den Fall der festen Färbepräparate isoliert man entweder bei Raumtemperatur durch Aussalzen und Filtrieren mit anschließender Trocknung oder trocknet direkt die erhaltene Synthesebrühe nach eventueller Zugabe von Einstell- bzw. Entstaubungsmittel.

Die Trocknung kann zum Beispiel in einem Umluftschrank, mit Schaufeltrockner, Walzentrockner, bevorzugt jedoch durch Sprühtrocknung mit einer Ein- oder Zweistoffdüse oder Zerstäuberscheibe erfolgen. Bei einigen Farbstoffen ist es vorteilhaft, daß man eine heiße Lösung der Komponente sprühtrocknet. Die Konzentration der Feststoffe in Wasser beträgt bei der Sprühtrocknung üblicherweise 10-45 Gew.-%.

Als Einstellmittel kommen übliche Einstellmittel, d. h. feste Verbindungen in Frage, die keine Reaktion mit der Reaktivgruppe eingehen wie z. B. anorganische Salze, besonders bevorzugt LiCl, $Li_2SO_4$ oder Polyphosphate, vorzugsweise aber Nichtelektrolyte wie Polyvinylpyrrolidon, Harnstoff, Salze wasserlöslicher aromatischer Sulfosäuren wie Benzolsulfonsäure und anionische Dispergiermittel wie sulfatierte primäre oder sekundäre aliphatische Alkohole mit 10 bis 18 Kohlenstoffatomen, sulfatierte Fettsäureamide, sulfatierte Alkylenoxyaddukte, sulfatierte partiell veresterte mehrwertige Alkohole, vor allem Sulfonate, wie langkettige Alkylsulfonate, beispielsweise Laurylsulfonat, Cetylsulfonat, Stearylsulfonat, Mersolate, Natriumdialkylsulfosuccinate, wie Natriumdioctylsulfosuccinat, Tauride, beispielsweise Oleylmethyltaurid (Natriumsalz), Alkylarylsulfonate, wie Alkylbenzolsulfonate mit geradkettiger oder verzweigter Alkylkette mit 7 bis 20 Kohlenstoffatomen und Mono- und Dialkylnaphthalinsulfonate, wie Nonylbenzolsulfonat, Dodecylbenzolsulfonat und Hexadecylbenzolsulfonat, sowie 1 Isopropyl-naphthalin-2-sulfonat, Di-iso-propylnaphthalinsulfonat und insbesondere Kondensationsprodukt, aus aromatischen Sulfonsäuren und Formaldehyd wie Naphthalinsulfonsäure, Ditolylethersulfonsäuren oder Terphenylsulfonsäuren mit Formaldehyd und/oder Kondensationsprodukte aus Cyclohexanon, Formaldehyd und Bisulfit und/oder Ligninsulfonate. Weiterhin können noch übliche Entstaubungsmittel wie Öle, Öl/Emulgator-Mischungen oder Phthalsäureester zugesetzt werden.

Die nach dem erfindungsgemäßen Verfahren hergestellten salzarmen festen Präparationen der Reaktivfarbstoffe haben vorzugsweise folgende Zusammensetzung :

Der Reaktivfarbstoffgehalt in der erfindungsgemäß hergestellten Präparation liegt bei 20-90 Gew.-% (bevorzugt 30-80 Gew.-%) ; außerdem enthält die Einstellung bis zu 30 Gew.-%, bevorzugt $\leq$ 20 Gew.-% Salz, bis zu 5 Gew.-% Puffersubstanzen für den pH-Wert 6,5-8,5, weiterhin gegebenenfalls 10-70 % eines konzentrationserhöhenden Zusatzstoffes, 0-50 % der üblichen Einstellmittel bzw. Mischungen derer, sowie bis zu 4 % Entstaubungsmittel.

Eine weitere bevorzugte Formierung der nach dem erfindungsgemäßen Verfahren hergestellten Reaktivfarbstoffe ist die konzentrierte Lösung bzw. auch Flüssigkristalleinstellung mit folgender Zusammensetzung :

Der Reaktivfarbstoffgehalt in der erfindungsgemäß hergestellten konzentrierten Lösung oder konzentrierten Flüssigkristalleinstellung liegt bei 8-40 Gew.-% (bevorzugt 10-30 Gew.-%) außerdem enthält die Flüssigeinstellung bis zu 2 Gew.-% Puffersubstanzen für den pH-Wert 6,5-8,5, bevorzugt 0,5-1 Gew.-% Puffer, gegebenenfalls bis zu 30 Gew.-% eines konzentrationserhöhenden Zusatzstoffes, sowie bis zu 10 Gew.-% Alkalisalze, bevorzugt $\leq$ 6 Gew.-% Lithiumsalze. Insbesondere bevorzugt sind die Flüssigeinstellungen.

Gegenstand der Erfindung ist weiterhin die Verwendung der über das erfindungsgemäße Verfahren hergestellten Reaktivfarbstoffe zur Zubereitung von Färbebädern und Druckpasten für die Anwendung auf natürlichen und synthetischen Substraten, insbesondere Baumwolle, Wolle, Regeneratcellulose, Papier und Leder.

## Beispiel 1

0,072 Mol 1-Hydroxy-3-sulfo-6-aminonaphthalin (I-Säure) werden in 50 ml Wasser mit $LiOH \cdot H_2O$ bei pH = 7 gelöst. In der Lösung werden 25 g ε-Caprolactam und 50 mg einer Fällungs-Kieselsäure mit einer Oberfläche von 300 $m^2/g$ gegeben. Mit 48 %iger Schwefelsäure wird der pH-Wert leicht gesenkt und dann wird nach Zugabe von Eis (T = 5 °C) mit 2,4,6-Trifluor-5-chlorpyrimidin kondensiert. Der pH-Wert wird mit einer Mischung aus $Li_2CO_3/Na_2CO_3$ im schwach sauren Bereich konstant gehalten. Ausbeute Kondensationsprodukt ca. 95 %.

Die Kondensation von I-Säure mit 2,4,6-Trifluor-5-chlorpyrimidin unter sonst gleichen Reaktionsbedingungen, aber ohne 50 mg der pyrogenen Kieselsäure als heterogener Katalysator läuft lediglich bis zu 60 % ab.

## Beispiel 2

0,085 Mol des sauren Azofarbstoffs der Formel (9)

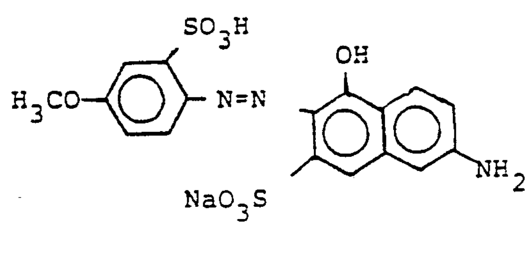

(9)

werden in 100 g Wasser mit $Li_2CO_3$ bei pH = 7 in Gegenwart von 70 g Harnstoff nahezu gelöst. Nach Zugabe von 90 g Eis und 250 mg einer Bleicherde (Tonsil®) mit einer Oberfläche von 230 $m^2/g$ als fester Katalysator wird mit 0,095 Mol 2,4,6-Trifluor-5-chlorpyrimidin in Gegenwart von weiterem $Li_2CO_3$ im schwach sauren bis neutralem Medium kondensiert. Nach ca. 5 h erhält man nach Aufarbeitung zu 95 % Umsetzungsprodukt.

Ohne Zusatz von Tonsil mit einer Oberfläche von 230 $m^2/g$ läuft die Umsetzung unter diesen Bedingungen lediglich zu 70 % ab.

## Beispiel 3

Anstelle von 250 mg Bleicherde (Tonsil®) mit einer Oberfläche von 230 $m^2/g$ in Beispiel 2 kann als heterogener Katalysator auch eine Mischung aus 300 mg Bleicherde (Tonsil®) mit Oberfläche von 240 $m^2/g$ mit 1 ml eines makroporösen $SO_3H$-Gruppen enthaltenen Kationenaustauschers auf Basis Styrol-Divinylbenzol (18 Gew.-%) eingesetzt werden. Man erhält in diesem Falle nach der Aufarbeitung eine Ausbeute von 85 % d. Theorie.

Beispiel 4

Anstelle von 250 mg Tonsil mit einer Oberfläche von 230 $m^2/g$ im Beispiel 2 werden als heterogener Katalysator 300 mg eines Mischoxides der Zusammensetzung 62 % $SiO_2$, 30 % MgO, 9 % $Al_2O_3$ und 3 % $Fe_2O_3$ mit einer Oberfläche von 0,2 $m^2/g$ zugegeben. Auch hier werden unter identischen Reaktionsbedingungen bis zu 95 % Umsetzungsprodukt (Reaktivfarbstoff) erhalten.

Beispiel 5

0,04 Mol des sauren Azofarbstoffs der Formel (10)

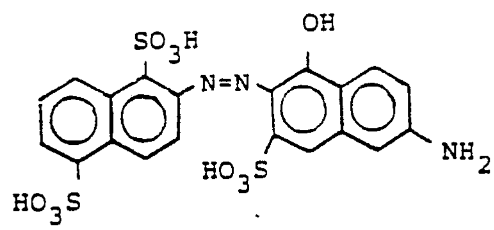

(10)

werden in 70 ml Wasser mit $Li_2CO_3$ bei pH = 5 nahezu gelöst. Nach Zusatz von 30 g Eis, 20 g Dimethylharnstoff und 160 mg einer Bleicherde (Tonsil®) mit einer Oberfläche von 240 $m^2/g$ wird mit 0,05 Mol 2,4,6-Trifluor-5-chlorpyrimidin in Gegenwart von weiterem $Li_2CO_3$ kondensiert. Man erhält bis zu 85 Gew.-% Umsetzungsprodukt. Ohne Zusatz von Bleicherde (Tonsil®) unter gleichen Reaktionsbedingungen erhält man lediglich $< 70$ % Umsetzungsprodukt.

Beispiel 6

1 Mol 1-Amino-8-hydroxynaphthalin-3,6-disulfonsäure (H-Säure) wird in 700 ml Wasser mit LiOH gelöst (pH-Wert 8). Nach Zugabe von 1 300 mg einer Fällungskieselsäure mit einer Oberfläche von 200 $m^2/g$ als heterogener fester Katalysator wird mit 1,05 Mol 2,4-Difluor-5-chlor-6-methylpyrimidin in Gegenwart von einem 1 : 1 Gemisch aus LiOH/NaOH bei pH 8 kondensiert. Das Umsetzungsprodukt aus 2,4-Difluor-5-chlor-6-methylpyrimidin und H-Säure (Ausbeute dieser Umsetzung : 98 % d. Th.) kann direkt als Kupplungskomponente zur Herstellung von Reaktivfarbstoffen verwendet werden.

Beispiel 7

0,3 Mol des sauren Azofarbstoffs der Formel (11)

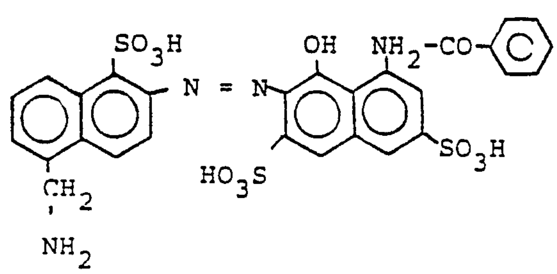

(11)

werden in 700 ml Wasser unter Zugabe von 3,25 Mol ε-Caprolactam nahezu gelöst. Als heterogener Katalysator werden 400 mg einer Fällungskieselsäure mit einer Oberfläche von 150 $m^2/g$ eingesetzt. Der Farbstoff wird anschließend mit 0,3 Mol 2,4,6-Trifluor-5-chlorpyrimidin in Gegenwart einer Mischung aus $LiOH \cdot H_2O/CaO$ bei pH 7-8 kondensiert. Man erhält so direkt eine lagerstabile Reaktivfarbstofflösung mit einer Ausbeute von $> 98$ % bezogen auf den Kondensationsschritt.

Beispiel 8

0,3 Mol des sauren Anthrachinonfarbstoffs der Formel (12)

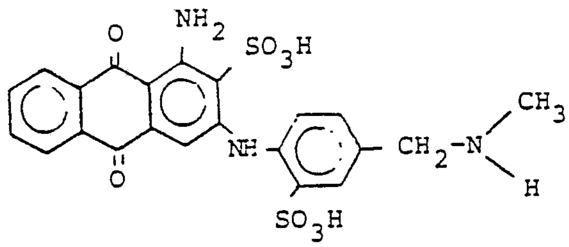

(12)

werden in 400 ml Wasser, 230 g ε-Caprolactam und 200 mg einer Fällungskieselsäure mit einer Oberfläche von ca. 240 m²/g suspendiert und mit 0,3 Mol 2,4,6-Trifluor-5-chlorpyrimidin in Gegenwart von $Li_2CO_3$ im schwach sauren bis neutralen Medium kondensiert. Man erhält bis zu 96 % Umsetzungsprodukt.

Beispiel 9

0,15 Mol des sauren Azofarbstoffs der Formel (13)

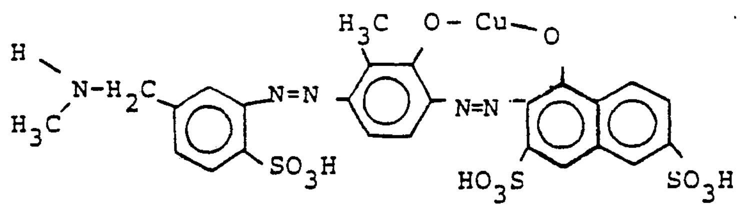

(13)

werden in 840 ml Wasser und 318 g ε-Caprolactam suspendiert. Nach Zugabe von 350 mg einer Fällungskieselsäure mit einer Oberfläche von 300 m²/g wird mit 0,155 Mol 2,4,6-Trifluor-5-chlorpyrimidin in Gegenwart von $Li_2CO_3$ im schwach sauren bis neutralen Medium kondensiert. Man erhält eine Reaktivfarbstofflösung, die nach Zusatz der für die Reaktivfärberei üblichen Hilfsmittel Baumwolle in marineblauem Ton färbt.

**Patentansprüche**

1. Verfahren zur Umsetzung von primären oder sekundären, aromatischen Zentren enthaltenden Aminen in wäßriger Lösung oder Suspension unter Zusatz von säurebindenden Mitteln bei pH 2-12 mit Verbindungen der Formel

$$R^1—X \qquad (1)$$

wobei
$R^1$ = heterocyclischer Reaktivrest und
X = Abgangsgruppe, bedeutet
dadurch gekennzeichnet, daß diese Umsetzung unter Zusatz reaktionsbeschleunigender heterogener Katalysatoren aus der Reihe der Metalloxide von Hauptgruppen- oder Übergangsgruppenelementen, Kieselgure, Kieselgele und Kieselsole, festen anorganischen Säuren und Basen des Titans und Wolframs, Oxide der Alkali- und Erdalkalimetalle auf Trägermaterialien, Bleicherden, Tonsile, des Talkums, Sande, Spinelle, synthetischer organischer Kationen- und Anionenaustauscher, Molekularsiebe und anorganischer Ionenaustauscher durchgeführt wird.

2. Verfahren gemäß Anspruch 1, dadurch gekennzeichnet, daß die heterogenen Katalysatoren eine Mindestoberfläche von 0,001 m²/g besitzen.

3. Verfahren gemäß Anspruch 1, dadurch gekennzeichnet, daß die Katalysatoren eine Mindestoberfläche von 0,1 m²/g besitzen.

4. Verfahren gemäß Anspruch 1, dadurch gekennzeichnet, daß die heterogenen Katalysatoren eine Mindestoberfläche von 10 m²/g besitzen.

5. Verfahren gemäß Anspruch 1, dadurch gekennzeichnet, daß man Verbindungen der Formel

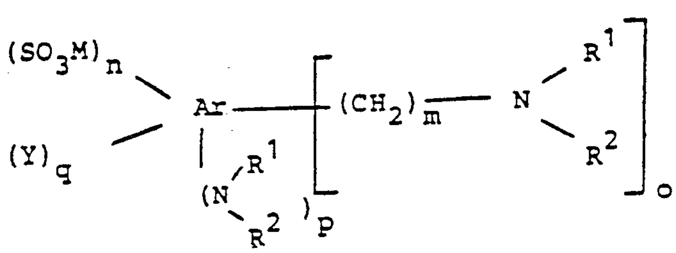

(3)

worin

Ar = Rest der Benzol- oder Naphthalinreihe oder eines chromophoren Systems der Mono-, Dis- oder Trisazoreihe, der Anthrachinon-, Oxazin- oder Formazanreihe oder der Reihe der Cu- oder Ni-Phthalocyaninfarbstoffe

Y = Substituent

M = Wasserstoff oder ein Metalläquivalent

$R^1$ = heterocyclischer Reaktivrest

$R^2$ = H oder $C_1$-$C_4$-Alkyl

m, q = ganze Zahl von 0 bis 6

n = ganze Zahl von 1 bis 5

o, p = 0 oder 1,

wobei o + p = 1

durch Umsetzung von Ausgangsverbindungen der allgemeinen Formel

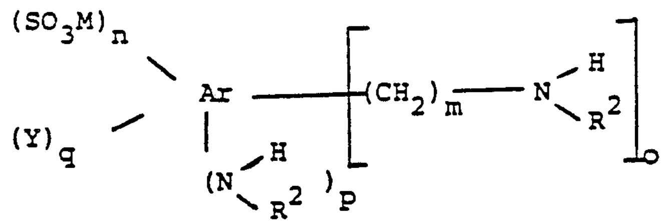

(2)

wobei M, $R^2$, Ar, Y, m, n, o, p, q die unter Formel (3) angegebene Bedeutung besitzen mit der heterocyclischen Reaktivkomponente der Formel

$$R^1—X \quad (3)$$

worin

$R^1$ = heterocyclischer Reaktivrest

X = Abgangsgruppe (vorzugsweise Cl oder F), herstellt.

6. Verfahren gemäß Anspruch 5, dadurch gekennzeichnet, daß als Verbindung der Formel (2) eine Hydroxyaminonaphthalinsulfonsäure oder ein Azofarbstoff auf Basis einer Hydroxyaminonaphthalinsulfonsäure als Kupplungskomponente eingesetzt wird.

7. Verfahren gemäß Anspruch 5, dadurch gekennzeichnet, daß als Verbindung der Formel (2) 1-Hydroxy-3-sulfo-6-aminonaphthalin (I-Säure) oder ein Azofarbstoff auf Basis von 1-Hydroxy-3-sulfo-6-aminonaphthalin eingesetzt wird.

**Claims**

1. Process for reacting primary or secondary amines containing aromatic centres with compounds of the formula

$$R^1—X \quad (1)$$

wherein

$R^1$ denotes a heterocyclic reactive radical and

X denotes a leaving group,

in aqueous solution or suspension at pH 2-12, with the addition of acid-binding agents, characterised in that this reaction is carried out with the addition of heterogeneous catalysts which accelerate the reaction from the series comprising the metal oxides of main group or transition group elements, kieselguhrs, silica gels and silica sols, solid inorganic acids and bases of titanium and tungsten, oxides of the alkali metals and alkaline earth metals on supports, bleaching earths, tonsils, talc, sands, spinels, synthetic organic cationic and anionic exchangers and molecular sieves and inorganic ion exchangers.

2. Process according to Claim 1, characterised in that the heterogeneous catalysts have a minimum surface area of 0.001 $m^2/g$.

3. Process according to Claim 1, characterised in that the catalysts have a minimum surface area of 0.1 $m^2/g$.

4. Process according to Claim 1, characterised in that the heterogeneous catalysts have a minimum surface area of 10 $m^2/g$.

5. Process according to Claim 1, characterised in that compounds of the formula

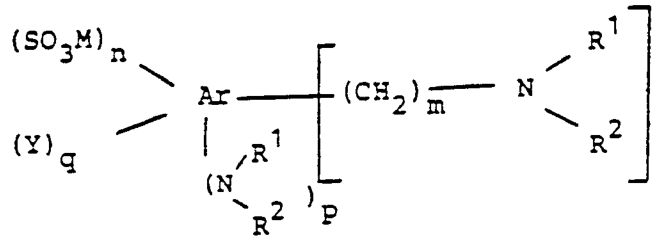 (3)

wherein

Ar = a radical of the benzene or naphthalene series or a chromophoric system of the mono-, dis- or tris-azo series, the anthraquinone, oxazine or formazan series or the Cu or Ni phthalocyanine dyestuff series,

Y = a substituent,

M = hydrogen or one equivalent of a metal,

$R^1$ = a heterocyclic reactive radical,

$R^2$ = H or $C_1$-$C_4$-alkyl,

m and q = integers from 0 to 6,

n = an integer from 1 to 5, and

o and p = 0 or 1,

wherein o + p = 1,

are prepared by reaction of starting compounds of the general formula

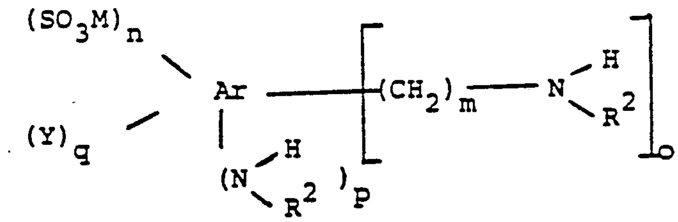 (2)

wherein M, $R^2$, Ar, Y, m, n, o, p and q have the meaning given under formula (3), with the heterocyclic reactive component of the formula

$$R^1—X \quad (1)$$

wherein

$R^1$ = a heterocyclic reactive radical and

X = a leaving group (preferably Cl or F).

6. Process according to Claim 5, characterised in that a hydroxyaminonaphthalenesulphonic acid or an azo dyestuff based on a hydroxyaminonaphthalenesulphonic acid as the coupling component is used as the compound of the formula (2).

7. Process according to Claim 5, characterised in that 1-hydroxy-3-sulpho-6-aminonaphthalene (I acid) or an azo dyestuff based on 1-hydroxy-3-sulpho-6-aminonaphthalene is used as the compound of the formula (2).

## Revendications

1. Procédé pour faire réagir une amine primaire ou secondaire contenant des centres aromatiques en solution ou suspension aqueuse, avec adjonction d'agents neutralisant les acides, à des pH de 2 à 12, avec des composés de formule

$$R^1—X \quad (1)$$

dans laquelle

$R^1$ représente un groupe réactif hétérocyclique, et

X représente un groupe éliminable,

caractérisé en ce que l'on effectue cette réaction avec adjonction de catalyseurs hétérogènes accélérant la réaction de la classe des oxydes métalliques des éléments des groupes principaux ou des groupes de transition, des kieselguhrs, des gels de silice et des sols de silice, des acides et bases minéraux solides du titane et du tungstène, des oxydes des métaux alcalins et alcalino-terreux sur matières de support, des terres de blanchiment, des Tonsils, du talc, des sables, des spinelles, des échangeurs de cations ou

d'anions organiques synthétiques, des tamis moléculaires et des échangeurs d'ions minéraux.

2. Procédé selon la revendication 1, caractérisé en ce que les catalyseurs hétérogènes ont une surface minimale de 0,001 $m^2/g$.

3. Procédé selon la revendication 1, caractérisé en ce que les catalyseurs ont une surface minimale de 0,1 $m^2/g$.

4. Procédé selon la revendication 1, caractérisé en ce que les catalyseurs hétérogènes ont une surface minimale de 10 $m^2/g$.

5. Procédé selon la revendication 1, caractérisé en ce que l'on prépare des composés de formule

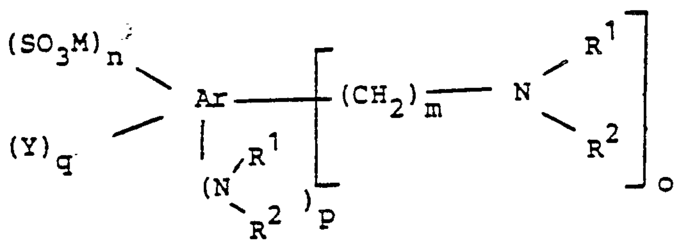

(3)

dans laquelle

Ar représente un radical de la série benzénique ou naphtalénique ou d'un système chromophore de la série mono-, dis- ou tris-azoïque, de la série anthraquinonique, oxazinique ou formazanique ou de la série des colorants de phtalocyanines de cuivre ou de nickel,

Y représente un substituant,

M représente l'hydrogène ou l'équivalent d'un métal,

$R^1$ représente un groupe réactif hétérocyclique,

$R^2$ représente H ou un groupe alkyle en $C_1$-$C_4$,

m et q sont des nombres entiers allant de 0 à 6,

n est un nombre entier allant de 1 à 5,

o et p sont égaux à 0 ou 1, et

$o + p = 1$,

par réaction de composés de départ de formule générale

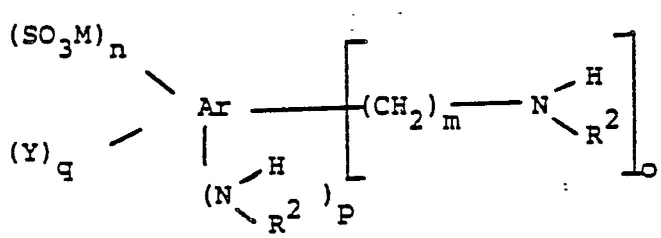

(2)

dans laquelle M, $R^2$, Ar, Y, m, n, o, p, q ont les significations indiquées en référence à la formule (3), avec le composant réactif hétérocyclique de formule

$$R^1—X \qquad (3)$$

dans laquelle

$R^1$ représente un groupe réactif hétérocyclique, et

X représente un groupe éliminable (en fait de préférence Cl ou F).

6. Procédé selon la revendication 5, caractérisé en ce que l'on met en œuvre en tant que composé de formule (2) un acide hydroxyaminonaphtalène-sulfonique ou un colorant azoïque à base d'un copulant consistant en un acide hydroxyaminonaphtalène-sulfonique.

7. Procédé selon la revendication 5, caractérisé en ce que l'on utilise en tant que composé de formule (2) le 1-hydroxy-3-sulfo-6-aminonaphtalène (acide I) ou un colorant azoïque à base du 1-hydroxy-3-sulfo-6-aminonaphtalène.